# EUROPEAN PATENT APPLICATION

(11) **EP 2 356 938 A1**
(43) Date of publication of application: **17.08.2011**
(21) Application number: 09825796.7
(22) Date of filing: 13.11.2009
(51) Int. Cl.: A61B 5/0476, A61B 5/0478

(54) **ELECTRODE FOR RECORDING BIOELECTROMAGNETIC SIGNALS AND RELATED MANUFACTURING PROCESS**

(30) Priority: 14.11.2008 ES 200803256
(71) Applicant: Consejo Superior de Investigaciones Cientificas (CSIC), 28006 Madrid (ES); Fundación del Hospital Nacional de Parapléjicos Para la Investigación y la Integración, 45071 Toledo (ES)
(72) Inventor: MORENO, Berta, E-28049 Madrid (ES); DEL CAMPO, Adolfo, E-28049 Madrid (ES); VILLANUEVA, Eugenio, E-28500 Arganda del Rey, Madrid (ES); CHINARRO, Eva, E-28049 Madrid (ES); JURADO, Jose Ramón, E-28049 Madrid (ES); GUINEA, Domingo, E-28500 Arganda del Rey, Madrid (ES); OLIVIERO, Antonio, E-45071 Toledo (ES); SOTO LEÓN, Vanesa, E-45071 Toledo (ES); FOFFANI, Guglielmo, E-45071 Toledo (ES)
(74) Representative: Pons Ariño, Angel
(86) International application number: PCT/ES2009/070501
(87) International publication number: WO 2010/055188

(57) **Abstract**

Electrode (1) for recording the bioelectromagnetic signals of a patient, that comprises: a flat matrix (2), that has an internal face (2a) and an external face (2b); at least one metal support (3) connected to the internal face (2a) of the flat matrix (2); at least one porous elastic part (4), impregnated with an inorganic electrically conductive dispersion, that has a fixing surface (4b) fixed to the metal support (3) and a contact surface (4a) to establish an electrical contact with the patient's skull.

## Description

### OBJECT OF THE INVENTION

The present invention belongs to the Electronic Chemistry area, within the field of bioelectric signal reception. Specifically, the object of the electrode of the invention is to record bioelectric signals of a patient without using contact gel.

### BACKGROUND OF THE INVENTION

Typically, a recording device of bioelectric activity comprises essentially a group of electrodes arranged in contact with the patient's skull at certain points, which are responsible for the acquisition of the signals. Subsequently, the signals are filtered for interpretation and / or storage. Since its invention in 1929, technological progress has mainly achieved, on the one hand, the transformation of the analogue signal into a digital signal, and secondly, the increase of the number of electrodes that can be used simultaneously. In recent years, thanks to signal analysis methods, electroencephalographic records (EEG) are used more and more in the diagnosis of neurological diseases, to study brain function and for the external control of devices.

Over the past years great progress has been made in EEG signal analysis: stationary spectral estimation methods to identify the main brain rhythms, dynamic spectral estimation techniques to correlate different EEG rhythms with the performance of sensorimotor and cognitive tasks; techniques to estimate the timing and directionality of different channels to characterize the transmission of activity between different areas of the brain; Laplacian spatial filtering methods to minimize the distortion due to the tissues that separate the electrodes in the brain; generator separation methods to locate the brain areas where neuronal activity originates, etc.

However, there are very few changes in the form and material of the electrodes used, still mostly being gold or Ag/Cl and in need of saline conductors (gel) at the point of contact with the patient's skin.

### DESCRIPTION OF THE INVENTION

In general, an electrode suitable for these applications must have a high electrical conductivity and good chemical stability, high mechanical strength, high flexibility and good dimensional stability. Two other key features are low cost and ease of recycling to avoid environmental damage.

The invention relates to an electrode that has a high electronic conductivity, good thermal and chemical stability (in the operating conditions). This is an organic-metal hybrid electrode that uses a polymer matrix of cross linked natural rubber latex. This gives it excellent mechanical properties, especially in elongation at break, a much higher flexibility than any electrode of the prior art, and an excellent elasticity. Also the weight and volume of the electrode of the invention are lower than those of the prior art. In addition, its cost is lower than the rest of the known electrodes, because, natural rubber latex in being an absolutely recyclable plant product, and of mass consumption, its price is several times lower than that of synthetic polymers derived from petrol used in other types of electrodes.

In this document, the term "patient" is intended to refer not only to human patients, but also any other animal whose brain activity could be studied.

A first aspect of the present invention discloses an electrode for recording bioelectric signals from a patient comprising a flat matrix, at least one metal support and at least one porous elastic piece. These elements are described hereunder:

### a) Flat matrix

A matrix whose approximately flat shape is suitable to adjust to the shape of the patient's skin on which the bioelectric signals will be taken. In a preferred embodiment of the invention, the flat matrix is made of an elastic material, but also can be made of rigid materials. To refer to the faces of the matrix, "inner face" shall refer to the side intended to be facing the patient's skin and "outer face" shall refer to the opposite side.

### b) Metal support

A piece of metal attached to the inside of the flat matrix, and is intended to support the porous elastic piece that acquires the bioelectrical signals of the patient's skin. The said bioelectrical signal is transmitted from the patient's skin through the porous elastic piece and the metal support, to a signal acquisition module or signal processing module. In a preferred embodiment of the invention, the electric board responsible for processing the bioelectrical signals captured is integrated into the outer face of the flat matrix.

In preferred embodiments of the invention, the metal support is made of copper shaped like a vessel, in order to house and secure the porous elastic part inside.

### c) The porous elastic piece

The porous elastic piece is the part of the electrode in contact with the patient's skin to acquire the bioelectric signal. The porous elastic piece is preferably made of natural latex, which can also be pre-vulcanized. To provide the porous elastic piece with electrical conductivity, it is mixed with an electrically conductive inorganic dispersion. The inorganic dispersion preferably comprises between 20% and 50% of metallic material, e.g. nickel, silver, gold, copper, aluminium, carbon, or others, and is mixed in the porous elastic piece to a concentration of preferably between 0, 1% and 1 % by weight.

The porous elastic piece can be any shape, provided that it is suitable for one side to be fixed to the metal support and the opposite side is suitable for contact with the patient's skin. In this paper, "attachment surface" refers to the surface that is attached to the metal support and "contact surface" refers to the surface which provides electrical contact with the patient. Preferably, the porous elastic piece is spherical, and more preferably with a diameter of between 500 microns and 5000 microns.

Furthermore, with the aim of further improving the conductivity of the porous elastic piece and electrical contact with the patient's skin, the contact surface of the porous elastic piece may also comprise a sheet of electrically conductive inorganic dispersion, preferably with a thickness of between 300 nm and 2 µm.

As for the mechanical properties of porous elastic pieces, regardless of the type of load used, the modules at 100% strain are less than 1.6 MPa, while at 900% they exceed in most cases 20 MPa, which means they are fairly elastic. All porous elastic pieces produced present a good performance against breaking, as the load at break varies between 15 and 30 MPa with maximum strain greater than 700%, well suited for the assembly under pressure from the porous elastic pieces on the metal supports.

Measurements have been taken of electronic conductivity of four points of the porous elastic pieces with a spherical shape and coated, and values of 10E⁴ S / cm at 25 ° C and 37 º C have been obtained, values that are very suitable for the recording of bioelectric signals.

On the other hand, the electrode manufacturing process of the invention of production is fast and simple, and requires no high temperatures or pressures, so it does not require much energy expenditure. Also, it does not require the use of any solvent, since all the dispersions are in aqueous phase, so it does not pollute.

Thus, a second aspect of the invention relates to a method of manufacturing an electrode for recording bioelectric signals of a patient, comprising a flat matrix, at least one metal support and at least one porous elastic piece, and which comprises the following steps:

### 1) Preparing a solution of an elastic material, preferably latex, and mixing it with an electrically conductive inorganic dispersion

Inorganic dispersion is prepared according to the type of inorganic / metal load. For example, the load can be ground in a ball mill with the required amount of water and surfactants. Another option is to prepare the inorganic dispersion using the sol-gel process, hydrolyzing a solution of a metal alkoxide base in an ethanol-water mixture.

### 2) Shaping the above mixture to obtain a porous elastic piece preferably spherical in shape.

A suitable treatment is applied to the mixture of inorganic dispersion with the solution of elastic material so that it solidifies in the desired shape.

### 3) Attaching the porous elastic piece to the metal support.

This process should result in addition to an appropriate mechanical attachment, a good electrical contact between the porous elastic piece and the metal support. Preferably, the attachment is made by thermo-junction at a temperature of between 40 ° C and 80 º C.

### 4) Attaching the metal support to a contact surface of the flat matrix.

Optionally a layer of electrically conductive inorganic dispersion can be added onto the contact surface of the porous elastic piece, preferably by screen printing, with the aim of further improving the electrical contact with the patient's skin.

Finally, another particular embodiment of the invention comprises the pre-vulcanization of the latex of the elastic porous piece to improve its mechanical properties.

### DESCRIPTION OF THE DRAWINGS

To complement the description being made and for the purpose of helping to better understand the features of the invention, according to a preferred practical embodiment thereof, a set of drawings is attached as an integral part of said description in which the following has been shown with an illustrative and non-limiting character:
Figure 1 Shows a view of a particular embodiment of an electrode according to the invention.
Figure 2 .- Shows an elastic flat matrix comprising a set of electrodes according to the invention.

### PREFERRED EMBODIMENT OF THE INVENTION

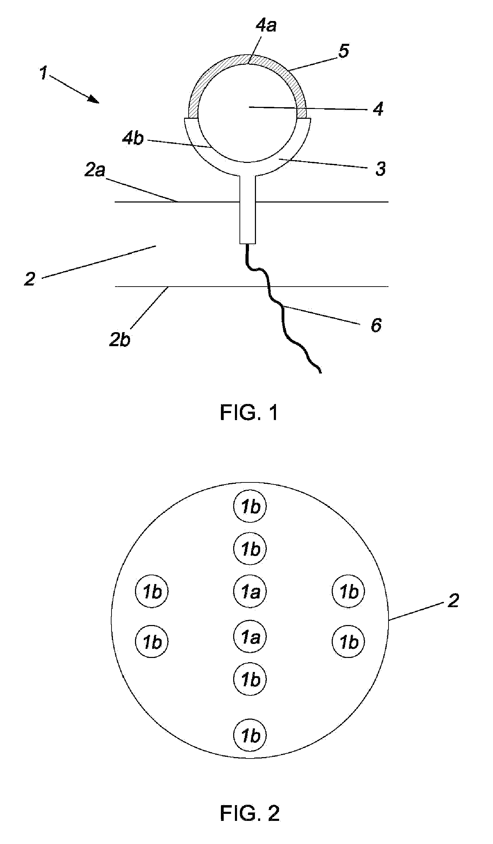

Hereunder is described a particular embodiment of the invention, referring to the attached figures. Figure 1 shows an electrode (1) according to the invention. The flat matrix (2) serves as the base for fixing the metal support (3), which in this example is made of copper shaped like a vessel, housing within a spherical porous elastic piece (4) formed from a mixture of inorganic dispersion that provides electrical conductivity and natural rubber latex dispersion.

The contact surface of the porous elastic piece (4) is coated with a layer (5) of inorganic colloidal dispersion, thus improving contact with the patient's skin. In this example, both the flat matrix (2) and the porous elastic piece (4) are made of pre-vulcanized natural latex.

Hereunder is described the manufacturing process used to manufacture the electrode (1), and whereby excellent mechanical properties have been achieved, especially the elongation at break, a much higher flexibility than that of all its competitors, and an excellent elasticity. Furthermore, recycling is easy, and it is not necessary to use solvents, so that the process is cheap and environmentally friendly. Thus, the steps used in manufacturing are:

### 1) Latex pre-vulcanization

First a dispersion of all reagents needed for cross linking the latex must be done: sulphur or sulphur donors, accelerators, antioxidants and associated surfactants and dispersants to help the formation and stability of the dispersion. To do so all the ingredients mixed with water at a rate less than 50% by weight are introduced into a ball mill and stirred for the time necessary to obtain an optimum particle size.

Subsequently the latex is pre-vulcanized, for which the dispersion of the vulcanization system agents is added and heated at between 30 and 70 ° C for 2-15 hours needed to achieve the desired degree of cross linking of the latex. Once done, the pre-vulcanized latex is suitably stabilized with the most appropriate surfactants to ensure its stability during the expected time of storage.

### 2) Preparation of the dispersion of the inorganic load.

The way to prepare the inorganic colloidal dispersion depends on the type of inorganic / metal load that is used. In the case of metals, the dispersion is prepared with a concentration of between 20 and 50% by weight grinding the load in a ball mill with the required amount of water and surfactants. The metal dispersions obtained by the sol-gel process are prepared by hydrolyzing a solution of base metal alkoxide in an ethanol-water mixture.

### 3) Mixture of the inorganic load with latex.

The pre-vulcanized latex is placed in a vessel fitted with mechanical agitation and the dispersion of the colloidal load is added gradually. Meanwhile, the mixing parameters are controlled such as viscosity, surface tension or pH to prevent clotting, for which various additives should be added to the mixture such as ionic and non-ionic surfactants or acids and bases.

### 4) Forming porous elastic pieces (4).

Pre-vulcanized latex is used and mixed with an electrically conductive inorganic colloidal dispersion to form porous elastic pieces (4) of a spherical shape. In this example, the diameter of the porous elastic pieces (4) is 500 microns.

### 5) Application of an inorganic colloidal dispersion sheet (5)

Finally, by using serigraphy a sheet (5) of the colloidal mixture is spread onto the contact surface of the spherical porous elastic pieces (4) at a temperature of 20-40 º C for less than 10 minutes, suitable for each colloidal mixture. Subsequently there is a drying process at 40 ° C which is essential for the coating to be free of fissures and cracking that could cause electrical disconnections.

### 6) Union of porous elastic pieces (4) with the metal supports (3).

Finally, the porous elastic pieces (4) are fixed to metal supports (3). In this example, the metal supports (3) are shaped like a vessel of 3000 microns in diameter and 500 microns deep, and are integrated on a printed circuit of signal acquisition (not shown in the figures) which is arranged on the outer face of a flat matrix (2).

The process of joining the metal support (3) / porous elastic latex piece (4) is carried out by a process of thermo junction at 60 °C which is essential for achieving the adhesion between these two materials.

### Detailed example

First a dispersion is prepared with the ingredients for pre-vulcanizing latex. To do this 100 g of sulphur, 50 g of zinc diethyldithiocarbamate, 20 g of zinc oxide, 20 g of antioxidant, 2 g of potassium caprylate, 2 g of casein and 200 g of water are introduced in a ball mill and stirred for two hours. To prevulcanize the latex, 500 g of latex concentrate (55% by weight of rubber) is added to a tank fitted with a mechanical stirring system, along with the amount of dispersion, prepared as discussed above, required so that for every 100 parts of rubber there are 2 parts of sulphur. To stabilize the mixture 10 g of aqueous 10% potassium caprylate and 10 g of Emulvin (a non-ionic surfactant) were added. The mixture was stirred at 200 rpm and heated for 6 hours at 60 º C.

To prepare the inorganic colloidal dispersion 1 g of colloidal metal solution and a few drops of surfactant Dolapix is added to a ball mill and stirred for 40 minutes. Then, using a container with a stirring system the mixing of the two solutions is carried out obtaining a blended unclotted mixture of both components. This mixture is formed into the metal supports (3) with a spherical shape, which in turn are attached to the elastic flat matrix and to a printed circuit provided on its outer face. Finally on the spherical porous elastic pieces (4) a surface coating is applied by serigraphy through a 100 micron sieve light mesh of a dispersion. Thus, it is achieved that the device is a compact whole without electrical disconnection. The electrode (1) is allowed to dry slowly for 5 hours at 40 º C to prevent cracking in the connections.

Thus clusters of electrodes have been prepared with a geometrical arrangement similar to that shown in Figure 2. In it, a pair of main measuring electrodes (1 a) arranged in the centre of the flat matrix (2) is surrounded by four pairs of electrodes (1 b) that serve as a filter to clean the signal of the central electrodes (1 a).

## Claims

1. Electrode (1) for the recording of a patient's bioelectric signals, which comprises:
a flat matrix (2), which has an inner side (2a) and an outer side (2b)
at least one metal support (3) fixed to the inner side (2a) of the elastic flat matrix (2);
at least one porous elastic piece (4) mixed with an electrically conductive inorganic dispersion, which has an attachment surface (4b) fixed to the metal support (3) and a contact surface (4a) designed to establish electrical contact with the patient's skin.

2. Electrode (1) according to claim 1, **characterized in that** the flat matrix (2) is made of an elastic material.

3. Electrode (1) according to either of claims 2-3, **characterized in that** the electrically conductive inorganic dispersion comprises between 20% and 50% of metallic material.

4. Electrode (1) according to claim 3, **characterized in that** the metallic material is at least one of the following: nickel, silver, gold, copper, aluminium and carbon.

5. Electrode (1) according to either of claims 3-4, **characterized in that** the metallic material content of the porous elastic piece (4) is between 0.1 % and 1% by weight.

6. Electrode (1) according to any of the preceding claims, **characterized in that** it further comprises a sheet (5) of inorganic dispersion arranged on the contact surface (4a) of the porous elastic piece (4).

7. Electrode (1) according to claim 6, **characterized in that** the dispersion sheet (5) has a thickness of between 300 nm and 2 µm.

8. Electrode (1) according to any of the preceding claims, **characterized in that** the porous elastic piece (4) is vulcanized natural latex.

9. Electrode (1) according to any of the preceding claims, **characterized in that** the porous elastic piece (4) is spherical.

10. Electrode (1) according to claim 9, **characterized in that** the porous elastic piece (4) has between 500 microns and 5000 microns in diameter.

11. Electrode (1) according to any of the preceding claims, **characterized in that** the metal support (3) is made of copper.

12. Electrode (1) according to any of the preceding claims, **characterized in that** the metal support (3) is shaped like a vessel.

13. Electrode (1) according to any of the preceding claims, **characterized in that** there is also an electric plate attached to the outer face of the flat matrix (2).

14. Manufacturing process of an electrode (1) for the recording of a patient's bioelectric signals, comprising a flat matrix (2), at least one metal support (3) and at least one porous elastic piece (4), **characterized in that** it includes the following steps:
preparing a solution of an elastic material and mixing the solution with an electrically conductive inorganic dispersion;
forming the mixture of the above operation to obtain a porous elastic piece (4);
fixing an attachment surface (4a) of the porous elastic piece (4) to the metal support (3), and
fixing the metal support (3) to a contact surface (2a) of the flat matrix (2).

15. Process for manufacturing an electrode (1) according to claim 14, **characterized in that** the elastic material is latex.

16. Process for manufacturing an electrode (1) according to claim 14, **characterized in that** the porous elastic piece (4) is spherically shaped.

17. Process for manufacturing an electrode (1) according to any one of claims 14-16, **characterized in that** the operation to fix the porous elastic piece (4) to the metal support (3) is performed by thermo-junction at a temperature of between 40 º C and 80 º C.

18. Process for manufacturing an electrode (1) according to any of claims 14-17, **characterized in that** it further comprises the operation to extend by means of screen-printing a layer (5) of electrically conductive inorganic dispersion onto a contact surface (4a) of the porous elastic piece (4).

19. Process for manufacturing an electrode (1) according to any of claims 14-18, **characterized in that** it further comprises the prior operation of pre-vulcanizing the latex of the porous elastic piece (4).
